# EUROPÄISCHE PATENTSCHRIFT

(11) **EP 1 303 267 B1**
(45) Veröffentlichungstag und Bekanntmachung des Hinweises auf die Patenterteilung: **07.04.2004**
(21) Anmeldenummer: 01960499.0
(22) Anmeldetag: 13.07.2001
(51) Int. Cl.: A61K 31/197, A61K 9/70, A61P 17/00

(54) **DERMALES APPLIKATIONSSYSTEM FÜR AMINOLÄVULINSÄURE**
DERMAL FOR AMINO LAEVULINIC ACID
SYSTEME D'APPLICATION DERMIQUE POUR L'ACIDE AMINOLEVULINIQUE

(30) Priorität: 17.07.2000 DE 10034673
(43) Veröffentlichungstag der Anmeldung: 23.04.2003
(73) Patentinhaber: Photonamic GmbH & Co. KG, 20354 Hamburg (DE)
(72) Erfinder: LEE, Geoffrey, 91077 Neunkirchen am Brand (DE); SZEIMIES, Rolf-Markus, 93197 Zeitlarn (DE)
(74) Vertreter: UEXKÜLL & STOLBERG
(86) Internationale Anmeldenummer: PCT/EP2001/008131
(87) Internationale Veröffentlichungsnummer: WO 2002/005809

(56) Entgegenhaltungen:
- EP-A- 0 704 209
- WO-A-00/28971
- WO-A-95/05813
- WO-A-95/07077
- WO-A-98/30242
- WO-A-99/11604
- US-A- 5 520 905

## Beschreibung

Die vorliegende Erfindung betrifft ein dermales Applikationssystem für Aminolävulinsäure.

Die topische Verwendung von 5-Aminolävulinsäure (ALA) (δ-ALA) bei der Behandlung oberflächlicher Hauttumoren, insbesondere von Basaliomen, ist erstmals 1990 von Kennedy et al. (J. Photochem. Photobiol. B. 6 (1990) 143-148) beschrieben worden, wobei in erster Linie visuell erkennbare Tumoren lokal mit ALA in Kontakt gebracht werden. Dabei wird ALA selektiv von Tumor-Gewebe aufgenommen und angereichert, wodurch es nur dort zu einer vermehrten Porphyrinbildung und -konzentration führt, während das gesunde Gewebe im wesentlichen unbeeinflußt bleibt. Die Wirkung von ALA beruht auf der Stimulierung der körpereigenen Porphyrinbildung. Da das Porphyrin bei Bestrahlung stark fluoresziert, kann die ALA- bzw. Porphyrin-Anreicherung im Tumorgewebe zur Diagnose präkanzeröser und kanzeröser Läsionen sowie zur photodynamischen Therapie von Tumorerkrankungen ausgenutzt werden.

Der pharmazeutischen Formulierung von ALA-Präparaten sind durch die Instabilität von ALA in wässriger Lösung praktische Grenzen gesetzt. So erweist sich ALA bei sehr niedrigem pH-Wert als ausreichend stabil, mit zunehmendem pH-Wert sinkt die Stabilität aber stetig (vergl. Rodriguez et al., S.P.I.E. (Society of Photo-optical Instrumentation Engineers) 2371 (1995) 204-209). Eine frische ALA-Lösung weist beispielsweise bei einem etwa physiologischen pH-Wert von 8 nach knapp zwei Wochen nur noch etwa 10% unzersetzten Wirkstoff auf. Aus diesem Grund sind im Handel ALA-Fertigpräparate wie Lösungen und Salben nicht erhältlich, sondern sie müssen, ausgehend von reiner ALA, unmittelbar vor der Anwendung frisch zu bereitet werden und weisen dann eine sehr begrenzte Haltbarkeit auf, die typischerweise weniger als zwei Wochen beträgt.

EP 0 704 209 A1 betrifft ALA-enthaltende Zusammensetzungen, insbesondere in Form von Gelen, Emulsionen und dergleichen, mit den oben beschriebenen Nachteilen.

WO95/05813 und WO96/06602 offenbaren Zusammensetzungen zur dermalen Applikation von ALA, die eine vergleichsweise niedrige Freisetzungsgeschwindigkeit für den Wirkstoff aufweisen.

Aufgabe der vorliegenden Erfindung ist es daher, ein ALA-Präparat zur Verfügung zu stellen, das in Form einer fertigen Formulierung vorliegt und eine Lagerstabilität bei minimierter Zersetzung der ALA aufweist.

Die Aufgabe wird durch das dermale Applikationssystem der vorliegenden Erfindung gelöst. Bei diesem System handelt es sich um ein selbstklebendes Matrixsystem, dessen Polymermatrix kristalline Aminolävulinsäure im Teilchengrößenbereich kleiner ca. 200 µm enthält.

Im Rahmen der vorliegenden Erfindung wurde überraschenderweise festgestellt, daß eine schnelle Freigabe der ALA durch die Wahl der selbstklebenden Polymermatrix nicht nachteilig beeinflußt wird. Aufgrund des ausgewählten Kristallgrößenbereichs wird die Sedimentation des ALA-Kristalle verhindert und es herrscht eine homogene ALA-Verteilung in der Matrix vor.

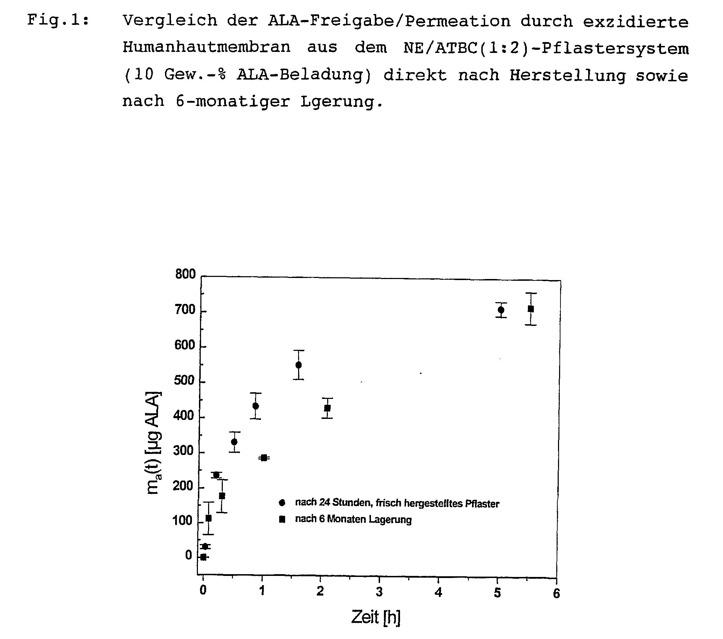

Als dermale Applikationssysteme kommen insbesondere die bislang bekannten Matrixsysteme des PSA-Typs (PSA: Pressure-Sensitive Adhesive) in Betracht, wie sie z.B. in Sugibayashi et al., J. Control. Rel. 29 (1994) 177-185 (vergl. insbesondere Figuren 1a und le), oder in der Monographie "Pharmazeutische Technologie: Moderne Arzneiformen" (Kapitel "*Transdermale Therapeutische Systeme*", M. Dittgen; Herausgeber: R. Müller, G. Hildebrand, Wissenschaftliche Verlagsgesellschaft Stuttgart, 1997) beschrieben sind.

Das erfindungsgemäße Applikationssystem enthält vorzugsweise eine wasserdurchlässige Polymermatrix, die besonders bevorzugt nur bedingt wasserdurchlässig ist.

Die selbstklebende Polymermatrix wird bevorzugt aus Polymeren aus der Gruppe bestehend aus
a) Acrylaten,
b) Silikonpolymeren und
c) Polyisobutylen
gebildet, die gegebenenfalls zusätzliche Weichmacher, wie z.B. Citronensäureestern (z.B. Acetyltributylcitrat, ATBC), enthält.

Bei der Wahl der Matrices sind Polymere bervorzugt, die gegenüber ALA nur eine geringes Lösungsvermögen aufweisen, wie z.B. Ethylacrylat-Methylmethacrylat-Copolymerisat (Eudragit NE). Von Vorteil ist ferner eine ausreichende Klebrigkeit, die die Herstellung von selbstklebenden Matrixsystemen ermöglicht, was durch Zusatz von Weichmachern (wie z.B. ATBC) erreicht werden kann.

Als selbstklebende Polymermatrix ist Eudragit NE (NE) mit Acetyltributylcitrat (ATBC) als Weichmacher besonders bevorzugt, insbesondere im Massenverhältnis NE/ATBC von 1:0,5 bis 1:2,5.

Im Rahmen der vorliegenden Erfindung hat sich gezeigt, daß ALA-Kristalle mit einem (mittleren) Durchmesser von weniger als 200 µm, vorzugsweise 20 bis 200 µm, besonders bevorzugt 30 bis 190 µm, von besonderem Vorteil sind. Am meisten sind ALA-Kristalle mit einem Durchmesser von 90 bis 160 µm bevorzugt.

Im erfindungsgemäßen dermalen Applikationssystem wird ALA vorzugsweise in einer Konzentration von bis zu 50 Gew.-%, insbesondere von mindestens 1 Gew.%, bezogen auf die fertige Polymermatrix eingesetzt. Besonders bevorzugt ist eine ALA-Konzentration von etwa 20 Gew.%.

Eine erfindungsgemäß besonders bevorzugte Ausführungsform der Erfindung betrifft ein Applikationssystem, bei dem ALA-Kristalle einen Durchmesser von 90 bis 160 µm besitzen und die Polymermatrix aus Eudragit NE (NE) und Acetyltributylcitrat (ATBC) im Gewichtsverhältnis NE/ATBC von 1:0,5 bis 1:2,5 besteht, wobei ALA in einer Konzentration von bis zu 50 Gew.-% bezogen auf die fertige Polymermatrix vorliegt.

Die Erfindung betrifft ferner ein Verfahren zur Herstellung dieses Applikationssystems, bei dem man gefriergetrocknetes Eudragit NE (NE) mit Acetyltributylcitrat (ATBC) in Aceton im Massenverhältnis NE/ATBC von 1:0,5 bis 1:2,5 auflöst, man anschließend gemahlener Aminolävulinsäure im Teilchengrößenbereich von 90 bis 160 µm in der Acetonlösung dispergiert und man die so erhaltene Dispersion zu einem dünnen Film auf einen Träger (eine Abdeckfolie) zieht, für 45 Minuten bei 60°C trocknet.

Das hiermit zur Verfügung gestellte Applikationssystem zeichnet sich insbesondere dadurch aus, daß ALA im Gegensatz zu Wirkstoffen herkömmlicher Pflastersysteme bzw. transdermaler Applikationssysteme sehr rasch freigesetzt wird und in die Haut eindringen kann. Die hohe Freisetzungsgeschwindigkeit war vor dem Hintergrund der bislang verfügbaren Daten und Kenntnisse auf dem Gebiet der transdermalen therapeutischen Systeme ebensowenig vorhersehbar wie die extrem hohe Lagerstabilität und die dadurch ermöglichte lange Aufbewahrungsdauer (d.h. Lagerfähigkeit bei minimaler Zersetzung von ALA).

Gemäß einer besonderen Ausführungsform der Erfindung werden durch das Applikationssystem innerhalb von 30 Minuten mindestens 30% der in der Polymermatrix dispergierten bzw. suspendierten ALA freigesetzt. Durch diese schnelle Wirkstoff-Freigabe kann die Einwirkzeit des Applikationssystems gegenüber herkömmlichen Applikationen mittels Salben oder Cremes verkürzt werden, d.h. die Verweilzeit des dermalen Applikationssystems ist deutlich geringer als die Anwendungsdauer bisher verwendeter ALA-haltiger Salben oder Cremes zur Applikation der gleichen Wirkstoffmenge. Gegenüber den erwähnten Salben oder Cremes weist das Applikationssystem ferner den Vorteil auf, daß ALA erzielt in einem scharf begrenzten Hautareal appliziert werden kann, während die bislang im Stand der Technik verwendeten Applikationsformen dies nicht ermöglichen und es daher auch zur Penetration umliegender Hautregionen kommt.

Mit dem dermalen Applikationssystem der vorliegenden Erfindung wird somit erstmals eine stabile, fertige Zubereitung von ALA zur Verfügung gestellt, die auch nach Lagerung über einen Zeitraum von einigen Wochen bis mehreren Monaten keine wesentliche Zersetzung von ALA aufweist. Wie überraschenderweise festgestellt wurde, bestehen bei dem erfindungsgemäßen System unmittelbar nach Herstellung' sowie nach sechsmonatiger Lagerung bei 25°C keine wesentlichen Unterschiede bezüglich der Wirkstoff-Freigabe und Hautpermeation *in vitro* oder *in vivo*.

Das genannte Applikationssystem ist somit zur Verwendung bei der photodynamischen Therapie und/oder Diagnose von präkanzerogenen oder kanzerogenen Läsionen der Haut, insbesondere von Hauttumoran (Basaliomen) besonders geeignet.

Die vorliegende Erfindung wird nachfolgend anhand von Beispielen beschrieben.

### BEISPIELE

### Beispiel 1

Herstellung eines erfindungsgemäßen dermalen Applikationssystems:

Die Pflasterherstellung kann mittels "Solvent Evaporation", "Hot Melt" oder anderer geeigneter Verfahren erfolgen (vgl. z.B. T. Peterson et al., "*Design, Development, Manufacturing and testing of Transdermal Drug Delivery Systems*" in "*Transdermal and Topical Drug Delivery Systems*"; T. Ghosh und W. Pfister, Ed.; Interpharm Press 1997, Buffalo Grove, IL/USA). Dies soll am Beispiel des "Solvent Evaporation"-Verfahrens veranschaulicht werden.

Gemäß einer Ausführungsform der Erfindung wird ALA erst gemahlen und klassifiziert, wobei der Teilchengrößenbereich 90 bis 160 µm verwendet wurde. Gefriergetrocknetes Eudragit NE (NE:Trägerpolymer) wurde zusammen mit Acetyltributylcitrat (ATBC; Weichmacher) in Aceton im Verhältnis NE/ATBC zwischen 1:0,5 und 1:2,5 aufgelöst. Anschließend erfolgte Zugabe und Dispersion von ALA in Konzentrationen in den fertigen Filmen bis zu 50 Gew.-% (% g/g). Die Zubereitung wurde anschließend zum dünnen Film auf eine Abdeckfolie gezogen und 45 Min. bei 60°C getrocknet. Als Abdeckfolie (bzw. Abziehfolie für die Seite des Films, die mit der Haut in Kontakt kommt) eigneten sich besonders Melinex 813 bzw. eine silikonisierte Abdeckfolie.

Die Filmklebrigkeit kann durch den Gehalt an ALA, durch das verwendete Polymer und den Anteil an Weichmacher (hier ATBC) variiert werden. Die ALA-Freigabe sowie deren Permeation durch intakte Haut wird durch ATBC beeinflußt (Weichmachereffekt bzw. Permeationsförderungseffekt).

Im Gegensatz zu den herkömmlichen transdermalen therapeutischen systemen (TTS) liegt ALA wegen deren hoher Hydrophilie bei allen Beladungen ≥ ca. 1 Gew.-% (% g/g) größtenteils suspendiert in der lipophilen NE/ATBC-Matrix vor.

### Beispiel 2

Die ALA-Partikel haben eine Größe zwischen ca. 90 und 160 µm und sind im Pflaster homogen verteilt.

Eine homogene Verteilung der ALA-Partikel im fertigen Pflaster erfordert eine Minimalisierung der Sedimentation der Partikel im flüssigen Polymer/Weichmacher/ALA-Zubereitung während der Pflasterherstellung. Dies erfolgt mittels Optimierung der Viskosität der Zubereitung durch Einstellung der Polymerkonzentration. Das Sedimentationsverhalten der ALA-Teilchen wird durch eine Erhöhung der Viskosität reduziert.

| Konzentration von NE[%g/g] in Lösung | Viskosität der Lösung [mPas] | Sinkgeschwindigkeit der ALA-Teilchen* in Lösung [µm/10s] |
|---|---|---|
| 12 | 446 | 396 |
| 18 | 2180 | 119 |
| 24 | 3510 | 3 |

| | | |
|---|---|---|
| * Siebfraktion 60-90 µm | | |

Eine angesetzte NE-Konzentration von ≥25% g/g gewährleistet eine minimale Sedimentationsgeschwindigkeit der ALA-Teilchen.

Für andere Polymermatrices, d.h. andere Polymere, können sich andere ALA-Partikelgrößen als vorteilhaft erweisen, die vom Fachmann gemäß den vorliegenden Informationen und Beispielen auf einfache Weise ermittelt werden können.

### Beispiel 3

ALA im Pflaster ist langzeitstabil.

Die ALA-Freigabe bzw. Hautpermeation aus dem Pflaster direkt nach Herstellung sowie nach 6-monatiger Lagerung bei 25°C zeigt keine wesentlichen Unterschiede (Fig. 1):

Zur Bestimmung des Freigabeprofils wird das Pflaster in eine Diffusionszelle nach Franz (vgl. z.B. K. Tojo, "*Designated Calibration of in vitro Permeation Apparatus*" in "*Transdermal Controlled Systemic Medication";* Y. Chien, Ed., Marcel Dekker, 1987) bei 33 °C eingespannt. Proben der wässrigen Akzeptorlösung werden nach verschiedenen Zeiten gezogen und deren ALA-Gehalt mittels Fluoreszenz/Derivatisierungs-HPLC-Methode bestimmt.

### Beispiel 4

Durch Anwendung eines Pflasters kann ALA homogen auf Hautläsionen aufgetragen werden.

Die extrem leichte Handhabung der Pflastersysteme stellt gegenüber Salbengrundlagen eine wesentliche Verbesserung für Arzt und Patient dar. Ein entsprechendes ALA-haltiges Pflaster kann auf das zu behandelnde Hautareal recht genau zugeschnitten werden. Dies vermindert die Mitbehandlung der umrandeten Hautfläche, die mit Pflaster nicht beklebt ist.

Durch die Applikation via Pflaster kommt es zu einer scharf begrenzten Applikation, ohne umliegende Hautregionen mit zu penetrieren.

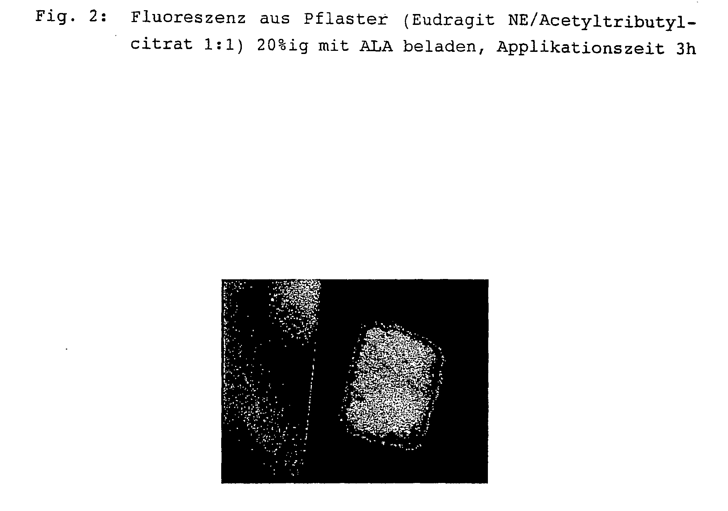

Nach 3h Applikation eines mit 20% ALA-beladenen Pflasters (Eudragit NE/Acetyltributylcitrat 1:1) auf Unterarm wird die Fluoreszenz der Hautareale gemessen. Fig. 2 zeigt, daß die Fluoreszenz scharf auf die Pflastergröße begrenzt ist und ein homogenes Aussehen hat.

### Beispiel 5

Überraschenderweise wird im Gegensatz zu herkömmlichen TTS ein hoher Anteil der Wirkstoffbeladung innerhalb kürzester Zeit freigesetzt.

Konventionelle TTS sind mit einer Vielfalt der eigentlich erforderlichen Dosis des Wirkstoffs beladen (Dittgen, M. *"Transdermale Therapeutische Systeme*" in "*Pharmazeutische Technologie; Moderne Arzneiformen*"; Müller, R. & Hildebrand, G., Ed.; Wissenschaftliche Verlagsgesellschaft Stuttgart, 1997). Diese überhohe Beladung ist notwendig, damit der Wirkstoff über einen Zeitraum von 1-7 Tagen annähernd mit konstanter Geschwindigkeit durch passive Diffusion freigesetzt wird. Während dieser Applikationszeit wird nur ein Anteil <50% der Gesamtwirkstoffbeladung freigesetzt.

Vergleichsbeispiel 1: Scopolamin TTS (Ciba) ist ein membrangesteuertes Pflaster und enthält insges. 1,5 mg Scopolamin. Es gibt 170µg Scopolamin/Tag ab und wird 3 Tage getragen. Am Ende des dritten Tages werden daher ca. 30% der Gesamtwirkstoffbeladung freigesetzt.

Vergleichsbeispiel 2: Estraderm TTS 25 (Geigy) ist ein klebstoffmembrangesteuertes Pflaster und enthält insges. 2 mg Estradiol. Es setzt 25 µg Estradiol/Tag frei und wird 3-4 Tage getragen. Während dieser Applikationszeit werden daher ca. 5% der Gesamtwirkstoffbeladung freigesetzt.

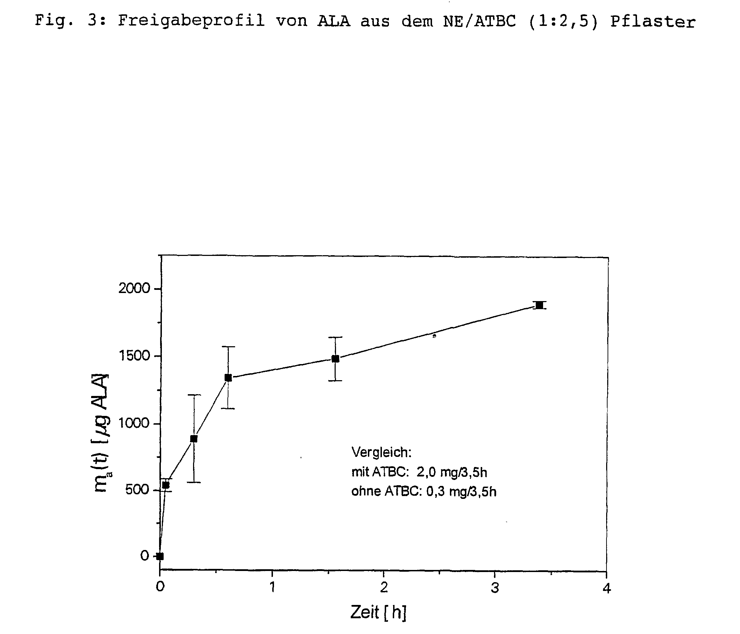

Im Gegensatz zu konventionellen TTS wurde überraschenderweise eine extrem rasche Freigabe der ALA aus dem NE/ATBC-Suspensinspflaster festgestellt. Fig. 3 zeigt das in vitro gemessene Freigabeprofil für ALA aus dem 250 µm-dicken Pflaster aus NE/ATBC (1:2,5) mit 20% g/g ALA-Beladung (Siebfraktion 90-160 µm). Das Pflaster enthielt ins. ca. 4 mg ALA/cm² und hatte bereits nach 1 Minute mehr als 500 µg ALA (entsprechend 12,5% der Gesamtwirkstoffbeladung) freigesetzt. Nach 30 Min. waren mehr als 1,3 mg ALA (entspricht 32 % der Gesamtwirkstoffbeladung) freigesetzt. Die Freigabeprofile wurden wie in Beispiel 3 beschrieben durchgeführt.

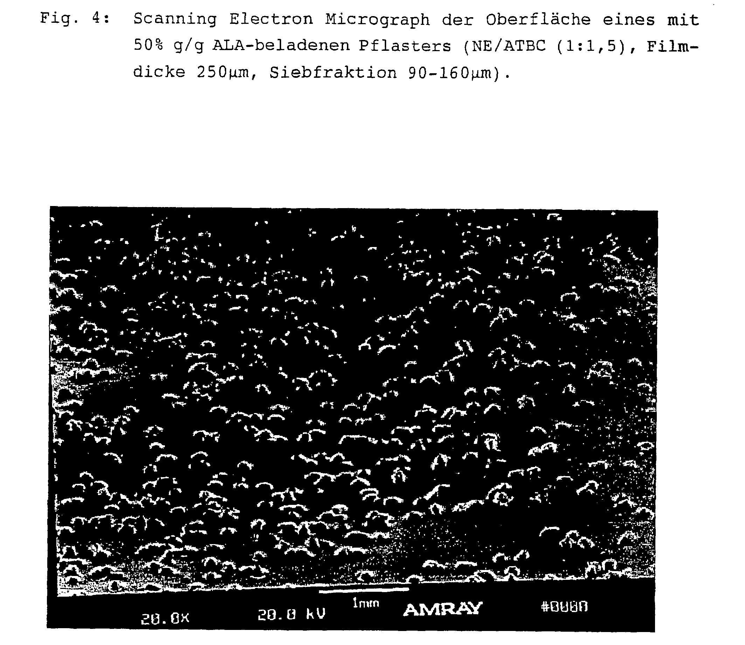

Der Grund für diese extrem rasche Freigabe ist auf den besonderen Aufbau bzw. die Morphologie des dermalen Applikationssystems (Suspensionspflasters) zurückzuführen. Bedingt durch die Anwesenheit suspendierter ALA in der NE/ATBC-Matrix ragen die 90 bis 160 µm großen ALA-Teilchen teilweise durch die Oberfläche der ca. 250 µm dicken Matrix heraus (vgl. Fig. 4).

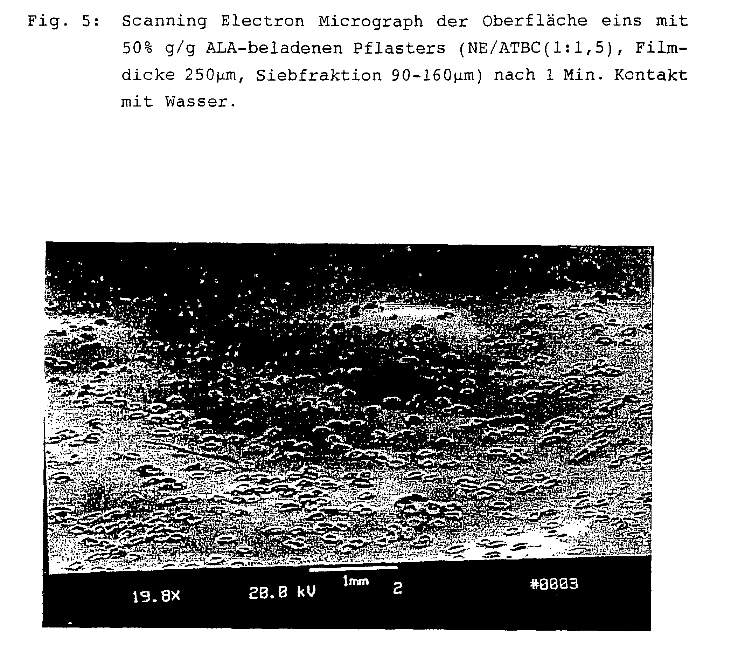

Nach kurzem Kontakt mit dem wässrigen Freigabemedium sind die durch die Oberfläche herausragenden ALA-Teilchen nicht mehr erkennbar (Fig. 5).

Diese unerwartet schnelle "Oberflächenauflösung" der ALA-Teilchen ist eine direkte Konsequenz ihrer hohen Hydrophile und führt zur beobachteten extrem raschen Freigabe der ALA aus dem Pflaster (vergl. Fig. 3).

### Beispiel 6

Die Einwirkzeit des Plastersystems für die Photodynamische Therapie (PDT) ist gegenüber anderen Applikationen mit Salben oder Cremes um ca. 30% verkürzt.

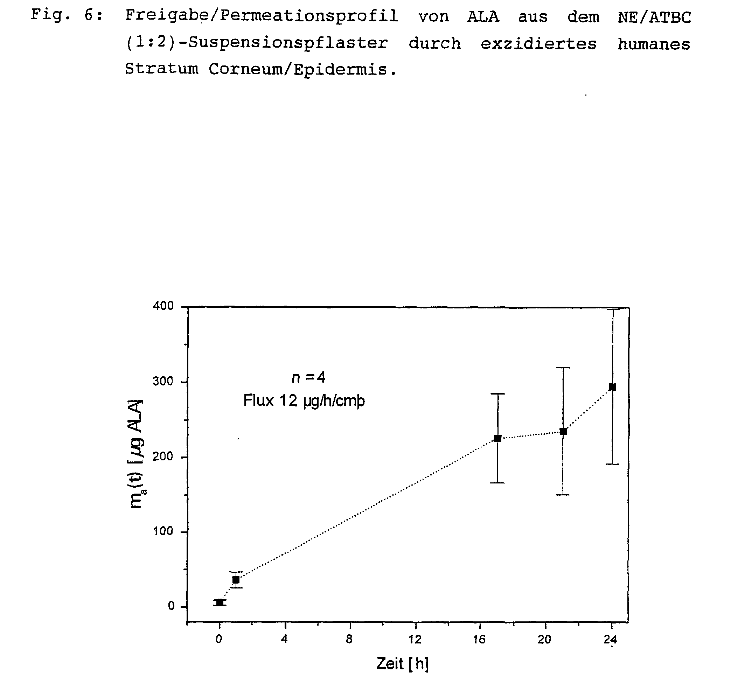

Bestimmung der Permeation von Wirkstoffen durch Membranen aus exzidiertem humanem Stratum Corneum/Epidermis kann mittels Franzzellen durchgeführt werden und stellt ein geeignetes Modell für die in vivo Aufnahme durch menschliche Haut dar. Fig. 6 zeigt das Freigabe/Permeationsprofil für ALA aus dem NE/ATBC (1:2,0)-Pflaster mit Filmdicke 250µm und beladen mit 20% ALA der Siebfraktion 90 bis 160 µm.

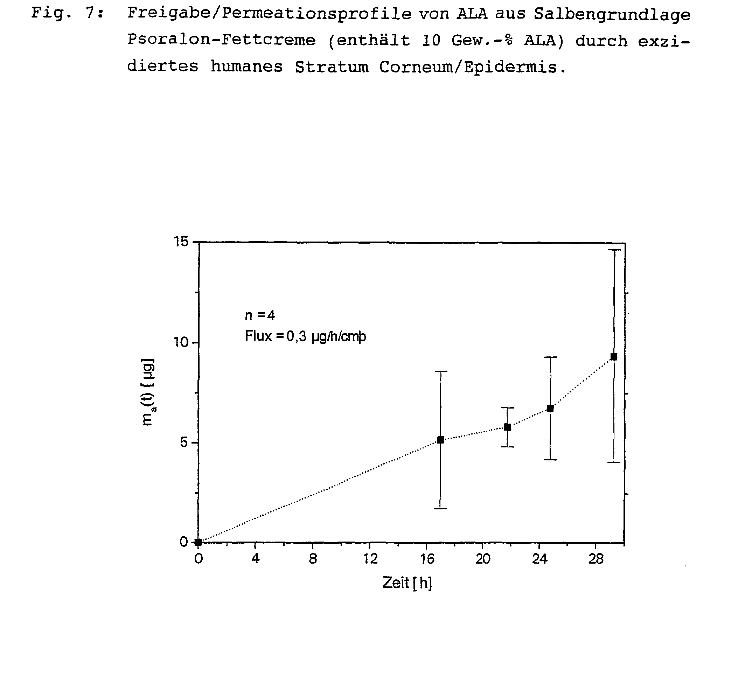

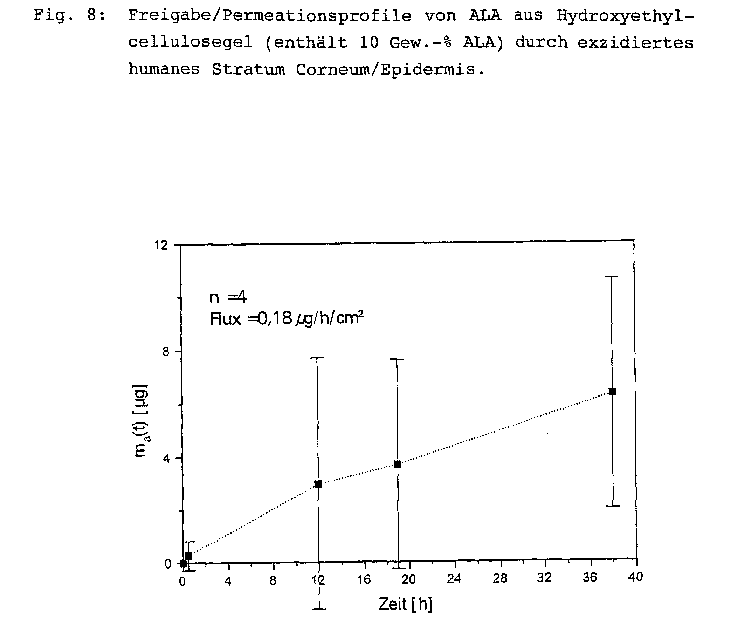

Nach 24 h haben bereits ca. 300 µg ALA die Humanhautmembran passiert. Im Vergleich dazu zeigen Fig. 7 und 8 die deutlich geringere Freigabe/Permeationsprofile der ALA aus den Salbengrundlagen Psoralon-Fettcreme® (enthält 10 Gew.-% ALA) und Hydroxyethylcellulosegel (enthält 10 Gew.-% ALA).

Aus beiden Salbengrundlagen haben nach 24 h weniger als 10µg ALA die Humanhautmembran passiert. Offensichtlich wird ALA aus dem Pflastersystem rascher und in wesentlich größeren Mengen durch die Humanhautmembran resorbiert. Ein Vergleich der Permeationsraten macht dies quantitativ deutlich:

| System/Grundlage | Permeationsrate [µg/cm²/h] |
|---|---|
| Pflaster NE/ATBC (1:2) | 12 |
| Psoralon-Fettcreme® | 0,3 |
| Hydroxyethylcellulosegel | 0,18 |

Eine raschere bzw. stärkere Wirkung des Pflasters gegenüber der Salbengrundlage während der in vivo PDT ist daher zu erwarten.

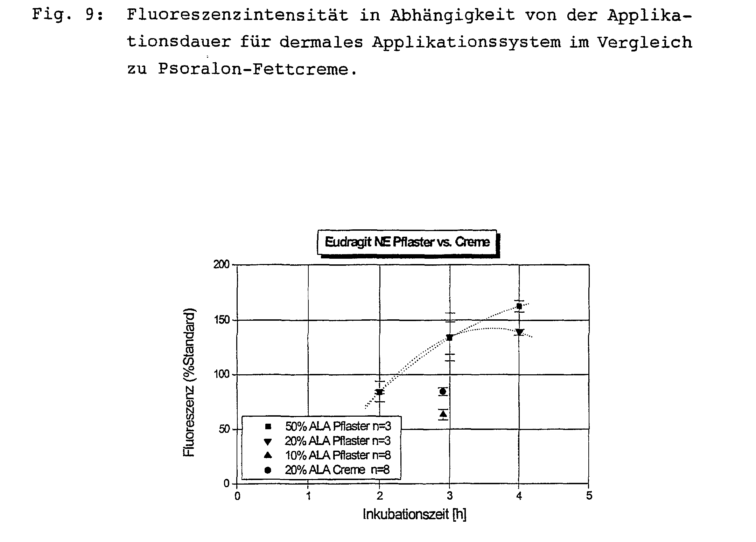

Fig. 9 zeigt die gemessene Fluoreszenzintensität an gesunden Probanden (Unterarm) in Abhängigkeit der Zeit. Die Intensität des mit 20%iger ALA beladenen NE/ATBC (1:2)-Pflasters betrug nach 2 h ca. 80% und nach 3 h ca. 140% einer Standardfluoreszenzzubereitung. Eine 50%ige ALA-Beladung des Pflasters gibt im Vergleich zur 20%igen Beladung der Pflaster keine weitere Steigerung in der Fluoreszenzintensität. Die gemessene Fluoreszenzintensität mit Psoralon-Fettcreme® (20% ALA-Beladung) nach einer Inkubationszeit von 3h ist deutlich niedriger (um ca. 60% als die aus dem NE/ATBC(1:2)-Pflaster (20% ALA-Beladung) bei gleicher Tragezeit (3h). Mit dem erfindungagemäßen Applikationssystem lassen sich somit deutlich kürzere Inkubations-(Applikationszeiten erzielen als mit Applikationsformen wie Salben oder Cremes.

## Patentansprüche

1. Dermales Applikationssystem, das ein selbstklebendes Matrixsystem ist, **dadurch gekennzeichnet, daß** die Polymermatrix kristalline Aminolävulinsäure (ALA) enthält, wobei die ALA-Kristalle eine Große von weniger als etwa 200 µm aufweisen.

2. Applikationssystem nach Anspruch 1, **dadurch gekennzeichnet, daß** die Polymermatrix wasserdurchlässig ist.

3. Applikationssystem nach den Ansprüchen 1 und 2, **dadurch gekennzeichnet, daß** die Polymermatrix aus Polymeren aus der Gruppe bestehend aus
a) Acrylaten,
b) Silikonpolymeren und
c) Polyisobutylen
ausgewählt ist.

4. Applilcationssystem nach den Ansprüchen 1 bis 3, **dadurch gekennzeichnet, daß** die Aminolävulinsäure-Kristalle einen (mittleren) Durchmesser von 30 bis 190 µm haben.

5. Applikationssystem nach Anspruch 4, **dadurch gekennzeichnet, daß** die Aminolävulinsäure-Kristalle einen (mittleren) Durchmesser von 90 µm bis 160 µm haben.

6. Applikationssystem nach den Ansprüchen 1 bis 5, **dadurch gekennzeichnet, daß** die Aminolävulinsäure in einer Konzentration von 1 bis 50 Gew.-% bezogen auf die fertige Polymermatrix vorliegt.

7. Applikationssystem nach den Ansprüchen 1 bis 6, **dadurch gekennzeichnet, daß** die Aminolävulinsäure-Kristalle einen Durchmesser von 30 bis 190 µm besitzen und die Polymermatrix aus Eudragit NE (NE) und Acetyltributylcitrat (ATBC) im Gewichtsverhältnis NE/ATBC von 1:0,5 bis 1:2,5 besteht, wobei Aminolävulinsäure in einer Konzentration von 1 bis 50 Gew.-% bezogen auf die fertige Polymermatrix vorliegt.

8. Applikationssystem nach Anspruch 7, **dadurch gekennzeichnet, daß** die Aminolävulinsäure-Kristalle einen Durchmesser von 90 bis 160 µm besitzen.

9. Applikationssystem nach den Ansprüchen 1 bis 8, **dadurch gekennzeichnet, daß** es innerhalb von 30 Minuten mindestens 30 % der Aminolävulinsäure freisetzt.

10. Applikationssystem nach den Ansprüchen 1 bis 9 zur Verwendung bei der photodynamischen Therapie und/oder Diagnose von präkanzerogenen und kanzerogenen Läsionen der Haut.

11. Applikationssystem nach den Ansprüchen 1 bis 9 zur Verwendung bei der photodynamischen Therapie und/oder Diagnose von Basaliomen.

12. Verfahren zur Herstellung des Applikationssystems nach den Ansprüchen 1 bis 9, **dadurch gekennzeichnet, daß** man gefriergetrocknetes Eudragit NE (NE) mit Acetyltributylcitrat (ATBC) in Aceton im Verhältnis NE/ATBC von 1:0,5 bis 1:2,5 auflöst, man anschließend gemahlene Aminolävulinsäure im Teilchengrößenbereich von weniger als etwa 200 µm in der Acetonlösung dispergiert und man die so erhaltene Dispersion zu einem dünnen Film auf eine Abdeckfolie zieht, für 45 Minuten bei 60 °C trocknet.

## Claims

1. Dermal application system which is a self-adhesive matrix system, **characterized in that** the polymer matrix contains crystalline aminolaevulinic acid (ALA), with the ALA crystals having a size of less than about 200 µm.

2. Application system according to Claim 1, **characterized in that** the polymer matrix is water-permeable.

3. Application system according to Claims 1 and 2, **characterized in that** the polymer matrix is selected from polymers derived from the group consisting of
a) acrylates,
b) silicone polymers and
c) polyisobutylene.

4. Application system according to Claims 1 to 3, **characterized in that** the aminolaevulinic acid crystals have an (average) diameter of from 30 to 190 µm.

5. Application system according to Claim 4, **characterized in that** the aminolaevulinic acid crystals have an (average) diameter of from 90 µm to 160 µm.

6. Application system according to Claims 1 to 5, **characterized in that** the aminolaevulinic acid is present at a concentration of from 1 to 50% by weight based on the finished polymer matrix.

7. Application system according to Claims 1 to 6, **characterized in that** the aminolaevulinic acid crystals have a diameter of from 30 to 190 µm and the polymer matrix consists of Eudragit NE (NE) and acetyltributyl citrate (ATBC) in an NE/ATBC ratio by weight of from 1:0.5 to 1:2.5, with aminolaevulinic acid being present at a concentration of from 1 to 50% by weight based on the finished polymer matrix.

8. Application system according to Claim 7, **characterized in that** the aminolaevulinic acid crystals have a diameter of from 90 to 160 µm.

9. Application system according to Claims 1 to 8, **characterized in that** it releases at least 30% of the aminolaevulinic acid within 30 minutes.

10. Application system according to Claims 1 to 9 for use in the photodynamic therapy and/or diagnosis of precarcinogenic and carcinogenic lesions of the skin.

11. Application system according to Claims 1 to 9 for use in the photodynamic therapy and/or diagnosis of basal cell carcinomas.

12. Process for producing the application system according to Claims 1 to 9, **characterized in that** freeze-dried Eudragit NE (NE) and acetyltributyl citrate (ATBC) are dissolved in acetone in an NE/ATBC ratio of from 1:0.5 to 1:2.5, ground aminolaevulinic acid, in a particle size range of less than about 200 µm, is then dispersed in the acetone solution and the resulting dispersion is stretched into a thin film on a cover foil [lacuna] dried at 60°C for 45 minutes.

## Revendications

1. Système d'administration dermique, qui est un système de matrice autocollant, **caractérisé en ce que** la matrice polymère contient l'acide aminolévulinique (ALA) cristallin, où les cristaux de ALA présentent une taille inférieure à environ 200 µm.

2. Système d'administration selon la revendication 1, **caractérisé en ce que** la matrice polymère est perméable à l'eau.

3. Système d'administration selon les revendications 1 et 2, **caractérisé en ce que** la matrice polymère est choisie parmi les polymères du groupe consistant en :
a) les acrylates ;
b) les polymères de silicone, et
c) le polyisobutylène.

4. Système d'administration selon les revendications 1 à 3, **caractérisé en ce que** les cristaux d'acide aminolévulinique ont un diamètre (moyen) allant de 30 à 190 µm.

5. Système d'administration selon la revendication 4, **caractérisé en ce que** les cristaux d'acide aminolévulinique ont un diamètre (moyen) allant de 90 à 160 µm.

6. Système d'administration selon les revendications 1 à 5, **caractérisé en ce que** l'acide aminolévulinique est présent en une concentration allant de 1 à 50%, par rapport à la matrice polymère finale.

7. Système d'administration selon les revendications 1 à 6, **caractérisé en ce que** les cristaux d'acide aminolévulinique possèdent un diamètre allant de 30 à 190 µm et que la matrice polymère consiste en l'Eudragit NE (NE) et le tributylcitrate d'acétyle (ATBC) en un rapport pondéral NE/ATBC allant de 1:0,5 à 1:2,5, où l'acide aminolévulinique est présent en une concentration allant de 1 à 50% en poids, par rapport à la matrice polymère finale.

8. Système d'administration selon la revendication 7, **caractérisé en ce que** les cristaux d'acide aminolévulinique ont un diamètre (moyen) allant de 90 à 160 µm.

9. Système d'administration selon les revendications 1 à 8, **caractérisé en ce qu'**il libère en 30 minutes, au moins 30% de l'acide aminolévulinique.

10. Système d'administration selon les revendications 1 à 9, pour l'utilisation en thérapie photodynamique et/ou pour le diagnostic de lésions précancérogènes et cancérogènes de la peau.

11. Système d'administration selon les revendications 1 à 9, pour l'utilisation en thérapie photodynamique et/ou pour la diagnostic de basaliomes.

12. Procédé de préparation de systèmes d'administration selon les revendications 1 à 9, **caractérisé en ce que** l'on dissout l'Eudragit NE (NE) lyophilisé avec le tributylcitrate d'acétyle (ATBC) dans l'acétone en un rapport NE/ATBC allant de 1:0,5 à 1:2,5, que l'on disperse ensuite l'acide aminolévulinique broyé, avec un intervalle de granulométrie inférieur à environ 200 µm, dans la solution d'acétone, et que l'on étale la dispersion ainsi obtenue en un film fin sur une feuille de couverture, que l'on sèche pendant 45 minutes à 60°C.
